# EUROPEAN PATENT SPECIFICATION

(11) **EP 1 482 852 B1**
(45) Date of publication and mention of the grant of the patent: **14.02.2007**
(21) Application number: 03709313.5
(22) Date of filing: 24.02.2003
(51) Int. Cl.: A61B 19/00, A61F 2/26

(54) **PENILE PROSTHESIS IMPLANT INSERTION TOOL**
EINFÜHRUNGSINSTRUMENT FÜR EIN PENISPROTHESENIMPLANTAT
OUTIL D'INSERTION D'IMPLANT DE PROTHESE PENIENNE

(30) Priority: 22.02.2002 US 358791 P
(43) Date of publication of application: 08.12.2004
(73) Proprietor: AMS Research Corporation, Minnetonka, MN 55343 (US)
(72) Inventor: GEORGE, Stephanie, A., St. Louis Park, MN 55416 (US); GOHMAN, James, A., Plymouth, MN 55442 (US)
(74) Representative: Vossius & Partner
(86) International application number: PCT/US2003/005602
(87) International publication number: WO 2003/071970

(56) References cited:
- US-A- 4 244 370
- US-A- 5 484 450

## Description

### Field of the Invention

The invention relates generally to instruments for penile prosthesis implantation. More particularly, the invention relates to an insertion tool particularly useful for inserting the proximal end of an implantable cylinder intended for implantation in the corpus cavemosum.

### Background of the Invention

Implantable penile prostheses are available for treatment of erectile dysfunction, and various specialized tools exist for implanting such prostheses. A typical penile prosthesis includes at least one pair of cylinders that are each implantable in one of the corpus cavernosa and a pump external to the cylinder for pressurizing the cylinder. The pump is typically connected to the cylinder through a tubing near the proximal end of the cylinder.

Typical implantation tools, often provided to surgeons in kits, include tools for inserting the distal end of the cylinder, tools for measuring the proximal and distal ends of the cylinder, tools for sizing and tools for suturing the incision after implantation of the cylinder. Typically these tools have been made of stainless steel and are designed to be sterilized and reused repeatedly. Care must be taken to ensure that the surfaces of the tools remain smooth over time so as to avoid unnecessary injuries to the tissues at the implantation site.

US-A-5 484 450 describes an instrument for use in penile prosthesis implant surgery or similar operation, the implant including a pair of inflatable, generally cylindrical members made of silicone material and a fluid tube coupled to each cylindrical member. The instrument is constructed and arranged to prevent damage to a cylindrical member upon closing an incision by suturing, the incision being made for the purpose of inserting the cylindrical member into the corpus cavernosum of the penis or for accessing the cylindrical member within the penis. The instrument includes an elongated handle and an elongated tool extending from the handle. The tool includes a connecting portion connected to the handle and a distal tool portion extending from the connecting portion. The distal tool portion is in the form of an arcuate wall defining a smooth concave surface and a smooth convex surface opposite the concave surface. The arcuate wall includes surfaces defining a notch in a distal end which opens away from the handle.

US-A-4 244 370 describes a tool for positioning an implantable medical prosthetic device. The tool comprises a hollow barrel having an obturator slidably mounted therein and having a rounded forward end. A locating device is attached to the barrel to stabilize the obturator in the plurality of predetermined positions as it is moved along the bore of the barrel. A needle having suture material attached may be placed within the bore of the barrel. A slot is located along the length of the barrel to allow the suture material to exit from the side of the barrel. In practice, the tool may be inserted into a bodily structure such as one of the corpora cavemosa of a penis. The outer surface of the barrel is equipped with a plurality of markings that allow the user to gauge the depth that the barrel has been inserted into a bodily structure. After the tool is inserted, the obturator is slid along the barrel bore to force the needle out of the rounded forward end of the barrel. The needle is manually pulled out of the bodily structure. After the tool is withdrawn, suture material remains threaded through the bodily structure.

There are no widely used tools specifically designed to assist in the implantation of the proximal cylinder end of penile prosthesis. There is thus a continuing need for penile implantation tools of this type that are safe, easy to use and versatile. The invention disclosed herein is aimed at providing a tool that achieves one or more of these goals while having substantially fewer drawbacks of the conventional tools.

### Summary of the Invention

Generally, the invention provides a device for inserting a penile implant prosthesis through an incision into a corpus cavernosum. The device includes an elongated, rigid body having a tip portion and tail portion, the tip portion including two prongs extending in a tip direction and a center portion between the two prongs, the prongs and center portion defining a notch adapted to engage and exert a force along the tip direction on a corner where a cylinder portion and a pump tubing of the prosthesis join each other, the prongs and center portion further defining a recess on one side of the center portion, the recess adapted to at least partially receive the cylinder portion or the pump tubing.

The elongated body of the device defines a channel disposed along a substantial portion of the elongated body. The tail portion of the elongated body can be formed as a paddle adapted to be inserted between the cylinder and tissues near or at the incision to prevent puncturing of the cylinder when the incision is being sutured closed. The tail portion can also be a rounded tail end adapted to engage an end of the cylinder portion for pushing the cylinder portion into the corporal body.

The tip portion can extend either in a direction parallel to the longitudinal axis of the elongated body or form an angle with it, depending on the desired operation.

### Brief Description of the Drawings

Other objects and advantages of the invention will become apparent upon reading the following detailed description and upon reference to the drawings in which:
Figure 1 schematically illustrates a penile implant prosthesis the implantation of which the insertion tool of the invention is particularly useful;
Figure 2 schematically shows the plan view of the insertion tool according to one aspect of the invention;
Figure 3 schematically shows the longitudinal cross-section view of the insertion tool shown in Figure 2;
Figure 4 schematically shows the side view of the insertion tool shown in Figure 2;
Figure 5 schematically shows an axial cross-sectional view of the handle portion of the insertion tool shown in Figure 2;
Figure 6 schematically shows an axial cross-sectional view of the neck portion of the insertion tool shown in Figure 2;
Figure 7 schematically shows a more detailed view of the tip region of the insertion tool shown in Figure 2;
Figure 8 schematically shows a tip-end view of the insertion tool shown in Figure 2;
Figure 9 schematically shows the plan view of an insertion tool according to another aspect of the invention;
Figure 10 schematically shows the longitudinal cross-section view of the insertion tool shown in Figure 9;
Figure 11 schematically shows the side view of the insertion tool shown in Figure 9;
Figure 12 schematically shows an axial cross-sectional view of the handle portion of the insertion tool shown in Figure 9;
Figure 13 schematically shows an axial cross-sectional view of the neck portion of the insertion tool shown in Figure 9;
Figure 14 schematically shows the plan view of an insertion tool according to another aspect of the invention;
Figure 15 schematically shows the longitudinal cross-section view of the insertion tool shown in Figure 14;
Figure 16 schematically shows the side view of the insertion tool shown in Figure 14;
Figure 17 schematically shows the tail-end view of the insertion tool shown in Figure 14
Figure 18 schematically shows an axial cross-sectional view of the handle portion of the insertion tool shown in Figure_ 14;
Figure 19 schematically shows an axial cross-sectional view of the neck portion of the insertion tool shown in Figure 14;
Figure 20 schematically shows a more detailed view of the tip region of the insertion tool shown in Figure 14;
Figure 21 schematically shows a tip-end view of the insertion tool shown in Figure 14;

While the invention is susceptible to various modifications and alternative forms, specific embodiments thereof have been shown by way of example in the drawings and are herein described in detail. It should be understood, however, that the description herein of specific embodiments is not intended to limit the invention to the particular forms disclosed, but on the contrary, the intention is to cover all modifications, equivalents, and alternatives falling within the scope of the invention as defined by the appended claims.

### Detailed Description of Specific Embodiments

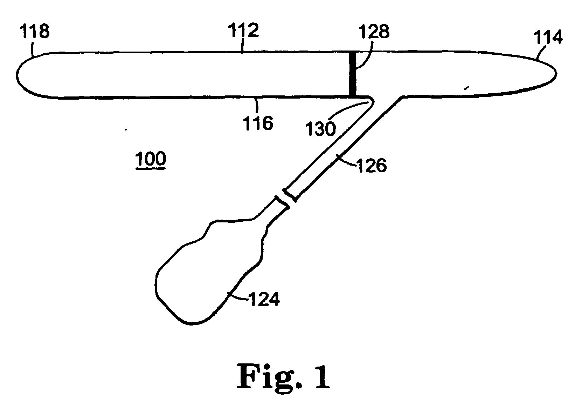

Referring to Figure 1, a penile implant prosthetic device 100, for the insertion tool of the invention is particularly useful, includes a cylinder or tubular enclosure 112, implantable in a corpus cavernosum, a pump bulb 124, to be disposed outside corpus cavernosum (typically implanted in the scrotum), and a tubing 126 connecting the cylinder 112 and the bulb 124. The cylinder 112 includes a proximal portion 114, a medial portion 116 and a distal portion 118. The medial portion 116 is flexible and can be pressurized by fluid pumped from the bulb 124 via the tubing 126. The proximal portion 114 is substantially more rigid than the medial portion 116 and includes the junction 128 between the cylinder 112 and tubing 126. The cylinder 112 and tubing 126 typically form a corner 130 that has an acute angle.

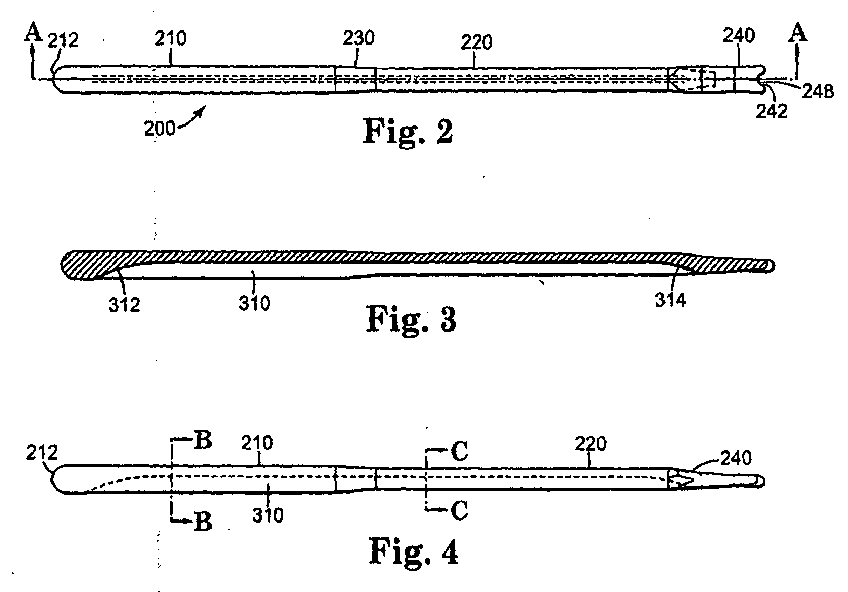

Referring to Figures 2 through 8, the insertion tool 200 in one embodiment of the invention has an elongated shape and includes a substantially round handle portion 210, a thinner, neck portion 220 having a U-shaped cross-section, a connecting portion 230 connecting the neck portion 220 to the handle portion 210, and a tip portion 240. The tool 200 in this embodiment is about 22,01 cm (8,7") long and about 0.81 cm (0.32") across in the handle portion 210, but can be of other sizes depending on the particular application.

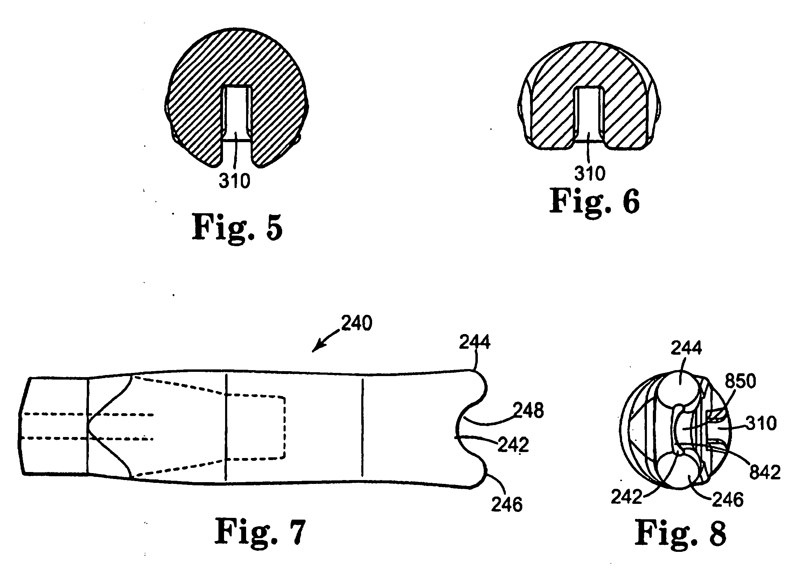

The tip portion 240 includes a center-portion 242 flanked by two round-edged prongs 244 and 246. Together, the center portion 242 and prongs 244, 246 define a round notch 248 with a diameter of about 0,38 cm (5.15"). Furthermore, the center portion 242 is thinner than the prongs 244, 246. The center portion 242 in this case also has a concave surface 842 (see Figure 8). The center portion 242 and prongs 244, 246 therefore define a recess 850 having a concave bottom 842. The recess 850 with the concave bottom 842 can be pushed against either the cylinder 112 or the pump tubing 126 when the tool 200 is used to insert the cylinder 120 in to the corpus body. The tip portion 240 extends substantially in the same direction as the handle and neck portions 210 and 220, enabling the implantation operation to be carried out with the tool 200 disposed at substantially the same angle when the recess 850 faces either way.

The insertion tool is made of molded plastic. The material and dimensions of the tool 200 are chosen so that the tool 200 is substantially rigid, *i.e.,* does not bend significantly under normal operating conditions for which the tool 200 is intended, or provides sufficient columnar strength to advance the proximal cylinder end within the corporal body.

The insertion tool 200 can also be partially or entirely coated with a layer of inert material that is compatible with both the penile prosthesis and human tissue, with both of which the tool 200 is likely to contact. For example, silicone or parylene can be applied to the tool 200 to produce a smoother surface, reducing the chances of injuring either the implant or tissue. The coated surface may also be more compatible with the surrounding tissue, reducing the probability of adverse reactions by the tissue. If the handle portion 210 is also coated, the coating can provide a more secure grip by the surgeon's gloved hand. The coating layer can be applied by a variety of known techniques, including simply dipping at least a portion of the insertion tool 200 in a suspension of the coating material and overmolding. The uncoated surfaces of the tool 200 can be deliberately roughened in preparation of coating to ensure superior bonding between the uncoated surfaces and the coating materials. Alternatively, one or more holes can be formed on the uncoated surfaces of the tool 200 so that dipped or overmolded material becomes mechanically attached to the tool 200 by one or more anchors formed in the hole(s).

The tail end 212 of the tool 200 in this embodiment is approximately semispherical with a radius of about 0,46 cm (0.18").

The handle portion 210, neck portion 220 and the connecting portion 230 of the tool 200 further define a channel 310 running through most of the length of the tool 200 and ending near the tail end 212 and the tip portion 240. The channel 310 in this case has substantially the same width, about 0.07", throughout most of its length. The channel 310 has tapered ends 312 and 314 to reduce the chances of channel 310 catching any tissue during operation. The channeled structure enhances the rigidity of the tool 200 and facilitates reduction or elimination of bubbles during the molding processing of the plastic. The channel 310 can also serve to at least partially receive the cylinder 112 or pump tubing 126, depending on which way the tip recess 850 faces, during the implantation procedure.

In operation, the tool 200 can be used to push the proximal portion 114 of the cylinder into the corporal body with the notch placed at the corner 130 between the cylinder 112 and tubing 126. The tool 200 can be positioned with the recess 850 conforming to either the cylinder 112 or pump tubing 126, depending on where the operating physician desires to apply pressure. In some cases, it may also be desirable to use the round tail end 212 to push the proximal portion 114 at the end where the flexible medial portion 116 is attached.

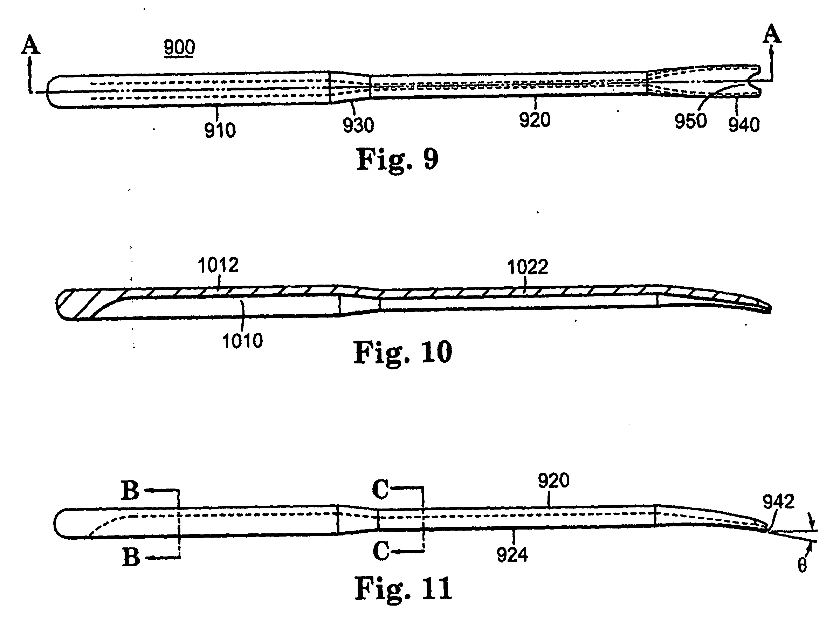

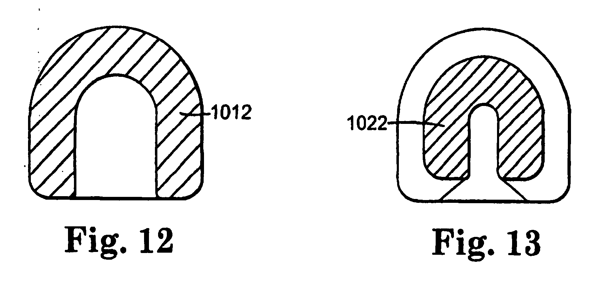

Referring to Figures 9 through 13, a second embodiment of the invention is an insertion tool 900, which is similar to the tool 200 in the example above but has a tip 940 that curves "up" towards the tip end 942 (with the direction of the opening of the channel 1010 being "up"). Thus, while in the previous embodiment the tip portion 240 extends no higher than the top surface of the neck portion 220, the tip end 942 is above the top surface 924 of the neck portion 920 by about 0.09". The tip portion in this case extends in a direction at an angle *θ* (see Figure 11) of about seven degrees from the longitudinal axis of the handle portion 910 and neck portion 920 but can be at other angles, such as between about five and ten degrees. This angled configuration results in a more secure engagement of the tool recess 950 with the cylinder 112 during the insertion operation as compared to a straight tip such as the tip 240 in the first embodiment.

The cross-sections of the handle portion 910, neck portion 920 and connection portion 930 are all substantially U-shaped in this illustrative embodiment. They collectively define a channel 1010 that has substantially the same thickness of the channel wall 1012 in the handle portion 910 as the channel wall 1022 in the neck portion 920. It is thought that having the same wall thickness throughout a molded plastic device may result in a more uniform plastic structure that is free of, or having significantly fewer, bubbles, voids and sink (or depression) spots, and offer improved columnar strength.

The tip portion 940 is also more streamlined than the tip portion 240 of the tool 200 in the previous example and can be more easily used as a suturing tool for protecting the cylinder from being punctured by the suturing needle when the incision is being closed after implantation of the cylinder. In such use, the concave surface of the tip is typically turned toward, and engaged to, the cylinder 112.

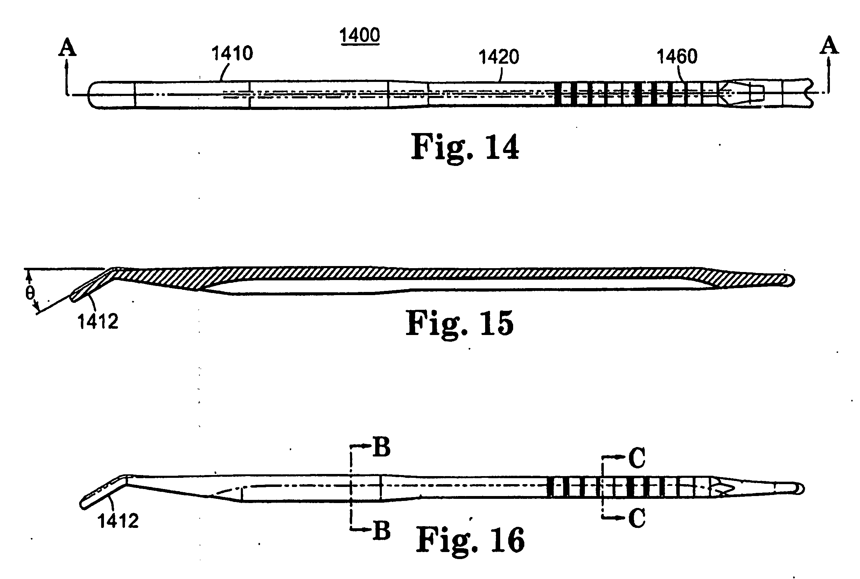

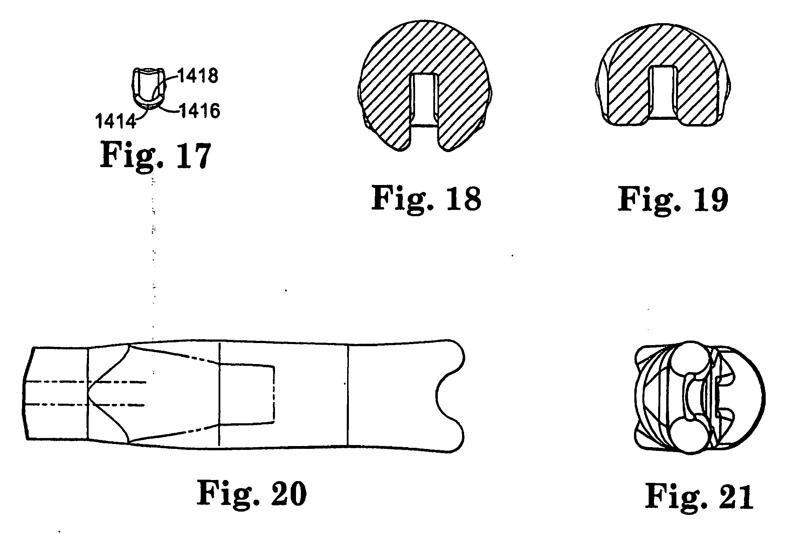

Referring to Figures 14 through 21, in a third illustrative embodiment of the invention, a insertion tool 1400 is similar to the tool 200 illustrated previously. However, instead having a round tail end 212, the tool 1400 has a tail segment 1412 that has a "downwardly"-curved wall 1414 having a concave "bottom" surface and convex "top" surface. Additionally, the tail segment 1412 in this embodiment extends in a direction at an angle *ϕ* of about 28 degrees from the longitudinal axis of the handle and neck portions 1410,1420, although other suitable angles, such as between about twenty and 35 degrees or between about fifteen and 45 degrees, can be used. The tail segment 1412 thus forms a paddle that makes the inserting tool 1400 also a closing tool, with the paddle 1412 used to protect the cylinder from being punctured by the suturing needle when the incision is being closed after implantation of the cylinder. Again, the concave surface is typically oriented to engage the cylinder surface 1418.

The insertion tool 1400 also includes several marks 1460 that are spaced apart from each other by predetermined distances. In this particular case the marks are about 0.394" (or 10 mm) apart. The marks serve as indicia of the depth of insertion. It should be noted that because the length of the proximal portion of the cylinder may be different in each patient's case, the marks 1460 are not indicative of the absolute depth but the distance from a predetermined reference point. The marks in this example are patterned such that each mark is visually distinct from its neighboring marks to facilitate easier tracking of the depth of insertion.

No limitations are intended to the details of construction or design herein shown, other than as described in the claims below. It is therefore evident that the particular embodiments disclosed above may be altered or modified and all such variations are considered within the scope of the claims. Accordingly, the protection sought herein is as set forth in the claims below.

## Claims

1. A device for inserting a penile implant prosthesis comprising a cylinder portion and a pump tubing, through an incision into a corpus cavernosum, the device comprising an elongated, rigid body having a tip portion (240) and tail portion, the tip portion including two prongs (244, 246) extending in a tip direction and a center portion (248) between the two prongs, the prongs and center portion defining a notch adapted to engage and exert a force along the tip direction on a corner where a cylinder portion and a pump tubing of the prosthesis join each other, the prongs and center portion further defining a recess on one side of the center portion, the recess adapted to at least partially receive the cylinder portion or the pump tubing, **characterised in that** the device is made of molded plastic and **in that** it is provided with a channel (310) disposed along a substantial portion of the elongated body*.*

2. The device of claim 1, wherein the tail portion of the body comprise a rounded tail end adapted to engage an end of the cylinder portion.

3. The device of claim 1, wherein the channel has two ends and a depth that diminishes gradually at both of the two ends.

4. The device of claim 1, wherein the added plastic body comprises polyetherimide.

5. The device of claim 1, further comprising an inert coating layer covering at least a portion of the elongated body.

6. The device of claim 5, wherein the inert coating layer covers substantially the entire elongated body.

7. The device of claim 5, wherein the inert coating layer comprises silicone.

8. The device of claim 1, wherein the elongated body is disposed substantially along a longitudinal axis, and the tip direction is substantially parallel to the longitudinal axis of the elongated body.

9. The device of claim 1, wherein the elongated body is disposed substantially along a longitudinal axis, and the tip direction forms an angle of at least five degrees with the longitudinal axis of the elongated body.

10. The device of claim 1, wherein the center portion has a concave surface forming a bottom of the recess.

11. The device of claim 1, wherein the tail portion of the elongated body comprises a paddle adapted to be inserted between the cylinder and tissues near or at the incision to prevent puncturing of the cylinder when the incision is being sutured closed.

12. The device of claim 11, wherein the elongated body is disposed substantially along a longitudinal axis, and the paddle extends in a direction at least twenty degrees from the longitudinal axis.

13. The device of claim 11, wherein the paddle has a concave surface that is adapted to be disposed to face the cylinder to prevent puncturing of the cylinder when the incision is being sutured closed.

## Patentansprüche

1. Vorrichtung zum Einführen einer Penisimplantatprothese mit einem Zylinderabschnitt und einer Pumpenröhrenanordnung durch eine Inzision in einen Schwellkörper, wobei die Vorrichtung einen langgestreckten, starren Körper mit einem Spitzenabschnitt (240) und einem Schwanzabschnitt aufweist, wobei der Spitzenabschnitt zwei Zinken (244, 246), die sich in einer Richtung der Spitze erstrecken, und einen Mittelabschnitt (248) zwischen den beiden Zinken hat, wobei die Zinken und der Mittelabschnitt eine Kerbe bilden, die dafür angepaßt ist, einzugreifen und entlang der Richtung der Spitze an einer Ecke, wo ein Zylinderabschnitt und eine Pumpenröhrenanordnung der Prothese sich vereinen, eine Kraft auszuüben, wobei die Zinken und der Mittelabschnitt ferner eine Aussparung an einer Seite des Mittelabschnitts definieren, wobei die Aussparung dafür angepaßt ist, den Zylinderabschnitt oder die Pumpenröhrenanordnung mindestens teilweise aufzunehmen, **dadurch gekennzeichnet, daß** die Vorrichtung aus geformtem Kunststoff besteht und daß die Vorrichtung mit einem Kanal (310) versehen ist, der entlang eines wesentlichen Abschnitts des langgestreckten Körpers angeordnet ist.

2. Vorrichtung nach Anspruch 1, wobei der Schwanzabschnitt des Körpers ein abgerundetes Schwanzende aufweist, das dafür angepaßt ist, mit einem Ende des Zylinderabschnitts in Eingriff zu treten.

3. Vorrichtung nach Anspruch 1, wobei der Kanal zwei Enden und eine Tiefe hat, die sich an beiden Enden allmählich verringert.

4. Vorrichtung nach Anspruch 1, wobei der geformte Kunststoffkörper Polyetherimid aufweist.

5. Vorrichtung nach Anspruch 1, ferner mit einer inerten Deckschicht, die mindestens einen Abschnitt des langgestreckten Körpers bedeckt.

6. Vorrichtung nach Anspruch 5, wobei die inerte Deckschicht im wesentlichen den gesamten langgestreckten Körper bedeckt.

7. Vorrichtung nach Anspruch 5, wobei die inerte Deckschicht Silikon aufweist.

8. Vorrichtung nach Anspruch 1, wobei der langgestreckte Körper im wesentlichen entlang einer Längsachse angeordnet ist und die Richtung der Spitze im wesentlichen parallel zur Längsachse des langgestreckten Körpers ist.

9. Vorrichtung nach Anspruch 1, wobei der langgestreckte Körper im wesentlichen entlang einer Längsachse angeordnet ist und die Richtung der Spitze einen Winkel von mindestens fünf Grad mit der Längsachse des langgestreckten Körpers bildet.

10. Vorrichtung nach Anspruch 1, wobei der Mittelabschnitt eine konkave Fläche hat, die einen Boden der Aussparung bildet.

11. Vorrichtung nach Anspruch 1, wobei der Schwanzabschnitt des langgestreckten Körpers einen Flügel aufweist, der zwischen dem Zylinder und Geweben nahe oder an der Inzision eingeführt werden kann, um eine Punktur des Zylinders zu verhindern, wenn die Inzision zugenäht wird.

12. Vorrichtung nach Anspruch 11, wobei der langgestreckte Körper im wesentlichen entlang einer Längsachse angeordnet ist, und der Flügel sich in einer Richtung mindestens zwanzig Grad von der Längsachse erstreckt.

13. Vorrichtung nach Anspruch 11, wobei der Flügel eine konkave Fläche hat, die dafür angepaßt ist, so angeordnet zu werden, daß sie dem Zylinder zugewandt ist, um eine Punktur des Zylinders zu verhindern, wenn die Inzision zugenäht wird.

## Revendications

1. Dispositif destiné à insérer un implant de prothèse pénienne comprenant une partie cylindrique et un tubage de pompage, par l'intermédiaire d'une incision dans un corps caverneux, le dispositif comprenant un corps rigide allongé comportant une partie de pointe (240) et une partie de queue, la partie de pointe comprenant deux griffes (244, 246) s'étendant dans une direction de la pointe et une partie centrale (248) entre les deux griffes, les griffes et la partie centrale définissant une encoche adaptée pour venir en prise et exercer une force le long de la direction de la pointe sur un coin où une partie cylindrique et un tubage de pompage de la prothèse se rejoignent, les griffes et la partie centrale définissant en outre un renfoncement sur un côté de la partie centrale, le renfoncement étant adapté pour recevoir au moins partiellement la partie cylindrique ou le tubage de pompage, **caractérisé en ce que** le dispositif est fait de plastique moulé et **en ce qu'**il est muni d'un canal (310) disposé le long d'une partie substantielle du corps allongé.

2. Dispositif selon la revendication 1, dans lequel la partie de queue du corps comprend une extrémité de queue arrondie adaptée pour venir en prise avec une extrémité de la partie cylindrique.

3. Dispositif selon la revendication 1, dans lequel le canal comporte deux extrémités et a une profondeur qui diminue graduellement aux deux extrémités.

4. Dispositif selon la revendication 1, dans lequel le corps en plastique moulé comprend du polyéthérimide.

5. Dispositif selon la revendication 1, comprenant en outre une couche de revêtement inerte recouvrant au moins une partie du corps allongé.

6. Dispositif selon la revendication 5, dans lequel la couche de revêtement inerte recouvre sensiblement tout le corps allongé.

7. Dispositif selon la revendication 5, dans lequel la couche de revêtement inerte comprend de la silicone.

8. Dispositif selon la revendication 1, dans lequel le corps allongé est disposé sensiblement le long d'un axe longitudinal, et la direction de la pointe est sensiblement parallèle à l'axe longitudinal du corps allongé.

9. Dispositif selon la revendication 1, dans lequel le corps allongé est disposé sensiblement le long d'un axe longitudinal, et la direction de la pointe forme un angle d'au moins cinq degrés par rapport à l'axe longitudinal du corps allongé.

10. Dispositif selon la revendication 1, dans lequel la partie centrale comporte une surface concave formant un fond du renfoncement -

11. Dispositif selon la revendication 1, dans lequel la partie de queue du corps allongé comprend un palpateur adapté pour être inséré entre le cylindre et les tissus près de ou sur l'incision pour empêcher la perforation du cylindre lorsque l'incision est suturée.

12. Dispositif selon la revendication 11, dans lequel le corps allongé est disposé sensiblement le long d'un axe longitudinal, et le palpateur s'étend dans une direction à au moins vingt degrés de l'axe longitudinal.

13. Dispositif selon la revendication 11, dans lequel le palpateur comporte une surface concave qui est adaptée pour être disposée en face du cylindre pour empêcher la perforation du cylindre lorsque l'incision est suturée.
